# EUROPEAN PATENT SPECIFICATION

(11) **EP 3 536 368 B1**
(45) Date of publication and mention of the grant of the patent: **17.09.2025**
(21) Application number: 19168470.3
(22) Date of filing: 09.11.2011
(51) Int. Cl.: A61M 15/00

(54) **DRY POWDER INHALER COMPRISING A SEROTONIN RECEPTOR AGONIST AND A DIKETOPIPERAZINE FOR TREATING MIGRAINES**
TROCKENPULVERINHALATOR MIT EINEM SEROTONINREZEPTORAGONIST UND EINEM DIKETOPIPERAZIN ZUR BEHANDLUNG VON MIGRÄNE
INHALATEUR DE POUDRE SECHE COMPRENANT UN AGONISTE DE RÉCEPTEUR DE SÉROTONINE ET UNE DICÉTOPIPÉRAZINE POUR LE TRAITEMENT DES MIGRAINES

(30) Priority: 09.11.2010 US 41177510 P; 10.11.2010 US 41233910 P
(43) Date of publication of application: 11.09.2019
(62) Divisional of application: 11788696.0
(73) Proprietor: MannKind Corporation, Danbury, CT 06810 (US)
(72) Inventor: Leone-Bay, Andrea, Ridgefield, CT 06877 (US); Stowell, Grayson, W., Gaylordsville, CT 06755 (US); Guarneri, Joseph, J., Stamford, CT 06905 (US); Carlson, Dawn M., Parrish, FL 34219 (US); Grant, Marshall, Newton, CT 06070 (US); Smutney, Chad, C., Watertown, CT 06795 (US)
(74) Representative: Potter Clarkson

(56) References cited:
- EP-A1- 1 598 066
- US-A1- 2009 111 749
- US-A1- 2009 308 390
- US-B1- 6 989 155

## Description

Methods and compositions for treating migraine are disclosed. The methods comprise administering a pharmaceutical formulation comprising a small molecule, including triptans such as sumatriptan to a patient in need of treatment using a drug delivery system for pulmonary inhalation. In particular, drug delivery systems comprising a breath powered dry powder inhaler for oral inhalation are described.

### BACKGROUND

Drug delivery systems for the treatment of disease which introduce active ingredients into the circulation for the treatment of disease are numerous and include oral, transdermal, subcutaneous and intravenous administration. While these systems have been used for quite a long time and can deliver sufficient medication for the treatment of many diseases, there are numerous challenges associated with these drug delivery mechanisms. In particular, delivery of effective amounts of proteins and peptides to treat a target disease has been problematic. Many factors are involved in introducing the right amount of the active agent, for example, preparation of the proper drug delivery formulation so that the formulation contains an amount of active agent that can reach its site(s) of action in an effective amount. Some examples of pulmonary inhalation delivery of drugs are known in the art, such as delivery of insulin in US2009/0111749 and sumatriptan in EP 1 598 066. US2009/308390 relates to a breath-powered, dry powder inhaler, a cartridge, and a pulmonary drug delivery system. US6989155 relates to use of particles of an amino acid in dry powder compositions.

Nevertheless, the active agent should be stable in the drug delivery formulation and the formulation should allow for absorption of the active agent into the circulation and remain active so that it can reach the target site(s) of action at effective therapeutic levels while minimizing the amount of dose to be administered. Thus, in the pharmacological arts, drug delivery systems which can deliver a stable active agent are useful.

### SUMMARY

The present invention relates to dry powder inhalers and is described in the following numbered paragraphs:
(1). A dry powder inhaler comprising an inhalable pharmaceutical composition comprising a dry powder comprising microparticles of 3,6-bis[(N-fumaryl-4-aminobutyl)]-2,5-diketopiperazine or a salt thereof, and a triptan selected from sumatriptan, almotriptan, eletriptan, frovatriptan, naratriptan, rizatriptan, zolmitriptan and pharmaceutically acceptable salts thereof, wherein the pharmaceutical composition further comprises an aliphatic amino acid selected from the group consisting of alanine, glycine, leucine, isoleucine, norleucine and serine.
(2). The dry powder inhaler of claim 1, wherein the triptan is present in an amount of 1-50 mg.
(3). The dry powder inhaler of claim 1 or claim 2, wherein the aliphatic amino acid comprises 0.5% to 30% by weight of the pharmaceutical composition.
(4). The dry powder inhaler of any one of claims 1-3, wherein the dry powder comprises microparticles, and 35% to 75% of the microparticles have an aerodynamic diameter of less than 5.8 µm.
(5). The dry powder inhaler of any one of claims 1-4, wherein the pharmaceutical composition further comprises L-leucine.
(6). A dry powder inhaler comprising an inhalable pharmaceutical composition comprising a dry powder comprising microparticles of 3,6-bis[(N-fumaryl-4-aminobutyl)]-2,5-diketopiperazine or a salt thereof, sumatriptan or rizatriptan, and 0.5% to 30% leucine or isoleucine.
(7). The dry powder inhaler of claim 6, wherein the sumatriptan or rizatriptan is present in an amount of 1-50 mg.
(8). The dry powder inhaler of any one of claims 1-7, wherein the 3,6-bis[(N-fumaryl-4-aminobutyl)]-2,5-diketopiperazine salt is an amorphous powder.
(9). The dry powder inhaler of any one of claims 1-8, wherein the 3,6-bis[(N-fumaryl-4-aminobutyl)]-2,5-diketopiperazine is a crystalline powder.
(10). The dry powder inhaler of any one of claims 1-9, wherein the rizatriptan is rizatriptan benzoate.
(11). The dry powder inhaler of any one of claims 1-10, wherein the pharmaceutical composition further comprises a pharmaceutically acceptable excipient.
(12). The dry powder inhaler of any one of claims 1-11, wherein the pharmaceutical composition further comprises a second medication or drug.
(13). The dry powder inhaler of claim 12, wherein the second medication or drug is fluoxetine or duloxetine.

Methods of introducing an active agent such as a serotonin receptor modulator, including serotonin receptor agonists or serotonin receptor antagonists, into the circulatory system of a mammal are disclosed herein. The methods comprise a drug delivery system which prevents deactivation or degradation of the active agent being administered to a patient in need of treatment. In particular, the drug delivery system is designed for pulmonary drug delivery such as by inhalation, for delivery of active agents to the pulmonary circulation in a therapeutically effective manner. In one option, the drug delivery system has advantages over other methods of drug delivery, for example, oral, subcutaneous and intravenous administration of drug products that are sensitive to enzymatic deactivation, or prevents other small molecules from degradation in the local peripheral and vascular tissue prior to reaching the target site.

There is disclosed herein, a method for providing an active agent to a patient in need thereof is disclosed comprising selecting an active agent subject to degradation in the patient wherein effectiveness of the active agent is reduced by the degradation; associating the active agent with a diketopiperazine to produce a pharmaceutical composition suitable for pulmonary inhalation; and providing the pharmaceutical composition to the patient so that the active agent reaches the target site with substantially no degradation or deactivation in therapeutically effective amounts at lower doses than standard dosing with other routes of administration.

Also disclosed herein is a method of treating a disease or condition comprising selecting a patient being treated with or a patient with a condition treatable by a labile active agent; providing a composition comprising the labile active agent in association with a diketopiperazine; and administering the composition to the patient via pulmonary inhalation; thereby treating the disease or condition.

In another option, a drug delivery system avoids degradation of the active agent from first pass metabolism, wherein the active agent is administered into the arterial circulation in the lungs and is delivered to the target organ in therapeutically effective levels, by avoiding degradation that occurs in venous blood circulation, in peripheral tissue, in the gastrointestinal system or in the liver. In this option, active agents can be delivered at lower concentrations than is required through other routes of administration. In a particular option, the active agent is a small molecule including, molecules that bind to serotonin receptors and are serotonin receptor agonists. In one option, the molecules induce vasoconstriction of blood vessels in the brain, relieve swelling and headaches. In
one option, the compositions are used for the treatment of moderate to severe headaches that interfere with a subject's performance of daily tasks, and showing symptoms of nausea, vomiting and sensitivity to light and noise.

In another option, the diketopiperazine is 2,5-diketo-3,6-di(4-X-aminobutyl) piperazine; wherein X is selected from the group consisting of succinyl, glutaryl, maleyl, and fumaryl; or a pharmaceutically acceptable salt thereof. In another option, the pharmaceutical composition is an inhalable dry powder formulation. In yet another option, the inhalable dry powder formulation further comprises a pharmaceutically acceptable carrier or excipient.

In one option, the inhalable dry powder formulation is provided to the patient by pulmonary inhalation using a dry powder inhalation system. In another option, the system comprises a dry powder inhaler with or without a container and a dry powder formulation.

There is described, a method for treating a migraine headache, which method comprises administering to a patient in need of treatment a dry powder composition by oral inhalation; wherein the dry powder composition comprises an active agent for treating migraines, including, a triptan such as sumatriptan, almotriptan, eletriptan, frovatriptan, naratriptan, rizatriptan, zolmitriptan and pharmaceutically acceptable salts thereof, and a substituted diketopiperazine such as fumaryl diketopiperazine, or a salt of the diketopiperazine such as disodium fumaryl diketopiperazine. The dry powder composition can be administered to the patient at the time of onset of the migraine headache as needed by the patient or as determined and instructed by the physician. In one option, the dose of the triptan can reduce or avoid unwanted side effects associated with injectable or tablet drug therapy, including, flushing, sweating, vertigo, fatigue, tingling, drowsiness, dizziness, dry mouth, heartburn, abdominal pain, abdominal cramps, weakness, feeling of warmth or coldness, bitter taste from tablets and nasal sprays, and local burning from injection site by proving a reduced amount of triptan required with other modes of administration.

There is described, a method of treating symptoms associated with migraine comprises administering to a subject in need of said treatment a therapeutically effective amount of a dry powder pharmaceutical composition by inhalation comprising a triptan, including, sumatriptan, almotriptan, eletriptan, frovatriptan, naratriptan, rizatriptan, zolmitriptan and pharmaceutically acceptable salts thereof, and a substituted diketopiperazine in a composition comprising bis[3,6-(N-fumaryl-4-aminobutyl)]-2,5-diketopiperazine or bis[3,6-(N-fumaryl-4-aminobutyl)]-2,5-diketopiperazine disodium salt. The pharmaceutical composition can comprise a dry powder comprising a pharmaceutically acceptable carrier or other inactive agents. The amount of triptans in the dry powder composition, for example, sumatriptan succinate can vary depending on the subject's requirements, for example, the triptan can be in amounts 1 mg or greater. The amount of sumatriptan or a salt thereof, including, sumatriptan succinate in a powder for pulmonary inhalation can be administered in a range of from about 1 mg to about 50 mg. In another option, the triptan is a salt of rizatriptan, including, but not limited to benzoate. The triptan salts can be, for example, almotriptan malate, frovatriptan succinate, eletriptan hydrobromide, and naratriptan hydrochloride. The dry powder composition can optionally comprise an amino acid such an aliphatic amino acid, for example, alanine, glycine, leucine, isoleucine, norleucine at amounts ranging from about 0.5% to about 30% by weight. In one option, the dry powder composition comprises the amino acid L-leucine. A pharmaceutical composition may comprise microparticles, wherein a microparticle may comprise 1) a diketopiperazine, and at least one of: a serotonin receptor agonist, such as a triptan, and an aliphatic amino acid. A triptan, and/or an aliphatic amino acid may be incorporated into, adhered to, complexed with, or coated onto a diketopiperazine microparticle. In some options, a diketopiperazine microparticle may be coated with at least one of a serotonin receptor agonist, such as a triptan, and an aliphatic amino acid.

There is disclosed herein, a method of treating a migraine headache which comprises a combination therapy administering a dry powder composition comprising a triptan by oral inhalation, and optionally, administering a second medication or drug, for example, selective serotonin reuptake inhibitor such as fluoxetine and duloxetine, which can be given by other routes of administration such as oral tablets or injections. The combination therapy can comprise a dry powder composition comprising the triptan and one or more additional drug(s) that can be administered by inhalation.

In another option of the disclosed method, the step of administering the composition to the patient comprises pulmonary administration of the dry powder
composition by inhalation using a breath powered, dry powder inhaler with or without a container, wherein the container can be a cartridge, such as a unit dosing cartridge for a reusable inhaler, or a single use inhaler. In this and other options, the dry powder inhaler system comprises a high resistance dry powder inhaler having air flow resistance values through its conduits in use of about 0.0065 to about 0.200 √(κPa)/L per minute, wherein the dry powder inhaler in use has an air flow distribution of from about 10% to about 30% through the container, which generates peak inhalation pressure differentials of about 2 κPa to about 20 κPa, and peak flow rates of between 7 L to about 70 L per minute.

### BRIEF DESCRIPTION OF THE DRAWINGS

FIG. 1 depicts an HPLC chromatogram of a solution containing sumatriptan-Na₂FDKP spray-dried powders.
FIG. 2A and B are scanning electron micrographs of three powders made by the present method comprising sumatriptan-Na2FDKP. Panels A and B represent powder particles without L-leucine. The powder particles with 10% leucine (Panel C and D) and the powder particles made with 20% leucine (Panel E and F).
FIG 3 depicts a graph of dose-normalized sumatriptan concentrations in blood samples following administration of sumatriptan-Na2FDKP by insufflation compared to sumatriptan administered by SC injection and sumatriptan nasal spray by instillation for a period of 4 hrs after administration.
FIG. 4 depicts a graph showing the mean sumatriptan levels in blood in dogs following sumatriptan administered as a nasal spray, sumatriptan administered intravenously and sumatriptan-Na2FDKP by insufflation compared to control dogs exposed to air insufflation.
FIG. 5 depicts a graph of pharmacodynamic data showing the effects of intravenously administered sumatriptan compared to Imitrex^{®} administered by nasal instillation and sumatriptan-Na2FDKP by insufflation on blood vessel diameter.

### DEFINITION OF TERMS

Prior to setting forth the invention, it may be helpful to provide an understanding of certain terms that will be used hereinafter:

Active Agents: As used herein "active agent" includes drugs, pharmaceutical substances and bioactive agents. Active agents can be small molecules, which are typically less than about 1,000 in molecular weight, do not necessarily have repeated units. Active agents can also be organic macromolecules including nucleic acids, synthetic organic compounds, polypeptides, peptides, proteins, polysaccharides and other sugars, and lipids. Peptides, proteins, and polypeptides are all chains of amino acids linked by peptide bonds. Peptides are generally considered to be less than 40 amino acid residues, but may include more. Proteins are polymers that typically contain more than 40 amino acid residues. The term polypeptide as is know in the art and as used herein, can refer to a peptide, a protein, or any other chain of amino acids of any length containing multiple peptide bonds, though generally containing at least 10 amino acids. The active agents can fall under a variety of biological activity classes, such as vasoactive agents, neuroactive agents, hormones, anticoagulants, immunomodulating agents, cytotoxic agents, antibiotics, antiviral agents, antigens, and antibodies. More particularly, active agents may include, in a non-limiting manner, insulin and analogs thereof, growth hormone, parathyroid hormone (PTH), ghrelin, granulocyte macrophage colony stimulating factor (GM-CSF), glucagon-like peptide 1 (GLP-1), and analogs of such peptides, alkynes, cyclosporins, clopidogrel and PPACK (D-phenylalanyl-L-prolyl-L-arginine chloromethyl ketone), antibodies and fragments thereof, including, but not limited to, humanized or chimeric antibodies; F(ab), F(ab)2, or single-chain antibody alone or fused to other polypeptides; therapeutic or diagnostic monoclonal antibodies to cancer antigens, cytokines, infectious agents, inflammatory mediators, hormones, and cell surface antigens.

Diketopiperazine: As used herein, "diketopiperazine" or "DKP" includes diketopiperazines, derivatives, analogs and modifications thereof, in both the salt and non-salt form of any of the foregoing, falling within the scope of the general Formula 1, wherein the ring atoms E1 and E2 at positions 1 and 4 are either O or N and at least one of the side-chains R1 and R2 located at positions 3 and 6 respectively contains a carboxylic acid (carboxylate) group. Compounds according to Formula 1 include, without limitation, diketopiperazines, diketomorpholines and diketodioxanes and their substitution analogs.

Diketopiperazines, in addition to making aerodynamically suitable microparticles, can also facilitate the delivery of active agents by rapidly dissolving at physiologic pH thereby releasing the active agent and speeding its absorption into the circulation. Diketopiperazines can be formed into particles that incorporate a drug or particles onto which a drug can be adsorbed. The combination of a drug and a diketopiperazine can impart improved drug stability. These particles can be administered by various routes of administration. As dry powders these particles can be delivered by inhalation to specific areas of the respiratory system, depending on particle size. Additionally, the particles can be made small enough for incorporation into an intravenous suspension dosage form. Oral delivery is also possible with the particles incorporated into a suspension, tablets or capsules.

In one option, the diketopiperazine is 3,6-di(fumaryl-4-aminobutyl)-2,5-diketopiperazine (fumaryl diketopiperazine, FDKP). The FDKP can comprise microparticles in its acid form or salt forms which can be aerosolized or administered in a suspension.

There is also disclosed where, the DKP is a derivative of 3,6-di(4-aminobutyl) 2,5-diketopiperazine, which can be formed by (thermal) condensation of the amino acid lysine. Exemplary derivatives include 3,6-di(succinyl-4-aminobutyl)-, 3,6-di(maleyl-4-aminobutyl)-, 3,6-di(glutaryl-4-aminobutyl)-, 3,6-di(malonyl-4-aminobutyl)-, 3,6-di(oxalyl-4-aminobutyl)-, and 3,6-di(fumaryl-4-aminobutyl)-2,5-diketopiperazine. U.S. Patent Nos. 5, 352,461, 5,503,852, 6,071,497, and 6,331,318, also describe examples of DKPs that may be used. The use of DKP salts is described in U.S. Patent No. 7,820,676. Pulmonary drug delivery using DKP microparticles is disclosed in U.S. Patent No. 6,428,771. Further details related to adsorption of active agents onto crystalline DKP particles can be found in U.S. Patent Nos. 7,799,344 and 7,803,404.

Drug delivery system: As used herein, "drug delivery system" refers to a system for delivering one or more active agents.

Dry powder: As used herein, "dry powder" refers to a fine particulate composition that is not suspended or dissolved in a propellant, carrier, or other liquid. It is not meant to necessarily imply a complete absence of all water molecules.

Percent respirable fraction per fill (%RF/Fill): As used herein "%RF/Fill" refers to the amount of powder particles emitted from an inhaler, or drug delivery system, which particles are in the respirable range and can be smaller than 5.8 µm, normalized by the total amount of powder filled into inhaler or drug delivery system. In some options, the inhaler comprises a cartridge for containing the dry powder.

Microparticles: As used herein, the term "microparticles" includes particles of generally 0.5 to 100 microns in diameter and particularly those less than 10 microns in diameter. Various options will entail more specific size ranges. The microparticles can be assemblages of crystalline plates with irregular surfaces and internal voids as is typical of those made by pH controlled precipitation of the DKP acids. In such options the active agents can be entrapped by the precipitation process or coated onto the crystalline surfaces of the microparticle. The microparticles can also be spherical shells or collapsed spherical shells comprising DKP salts with the active agent dispersed throughout. Typically, such particles can be obtained by spray drying a co-solution of the DKP and the active agent. The DKP salt in such particles can be amorphous. The forgoing descriptions should be understood as exemplary. Other forms of microparticles are contemplated and encompassed by the term.

Peripheral tissue: As used herein, "peripheral tissue" refers to any connective or interstitial tissue that is associated with an organ or vessel.

Potentiation: Generally, potentiation refers to a condition or action that increases the effectiveness or activity of some agent over the level that the agent would otherwise attain. Similarly it may refer directly to the increased effect or activity. Pulmonary inhalation: As used herein, "pulmonary inhalation" is used to refer to administration of pharmaceutical preparations by inhalation so that they reach the lungs and in particular options the alveolar regions of the lung. Typically inhalation is through the mouth, but in alternative options it can entail inhalation through the nose.

### DETAILED DESCRIPTION

There are disclosed herein, methods for the treatment of a disease or disorder utilizing a drug delivery system that can effectively deliver an active agent to the pulmonary circulation so that the active agent enters the pulmonary circulation and can be delivered in a therapeutic amount to the site(s) of action. The methods of treatment of disease or disorders comprise administering to a patient a formulation which can deliver the active agent directly or indirectly into the pulmonary circulation, and thereby to the arterial circulation, and can avoid degradation of the active agent by enzymes or other mechanisms in the local peripheral and/or vasculature tissues of the lungs. In one option, the method comprises the effective therapeutic delivery of active agents using a drug delivery system which allows for very rapid lung absorption of the active agent into the circulation and increases its effective bioavailability. In this option, lower dosages of an active agent can be delivered by this method of administration. In similar options effective doses can be achieved where they were not feasible by other modes of administration.

There is disclosed herein, a method for the treatment of disease. The inventors have identified the need to deliver drugs directly to the systemic circulation, in particular, the arterial circulation in a non-invasive fashion so that the drug reaches the target organ(s) prior to returning through the venous system. This approach may paradoxically result in a higher peak target organ exposure to active agents than would result from a comparable administration via an intravenous, subcutaneous or other parenteral route. A similar advantage can be obtained versus oral administration as, even with formulations providing protection from degradation in the digestive tract, upon absorption the active agent also enters the venous circulation.

The drug delivery system can be used with any type of active agent that is rapidly metabolized and/or degraded by direct contact with the local degradative enzymes or other degradative mechanisms, for example, oxidation, phosphorylation or any modification of the molecules including small molecules, proteins or peptides, in the peripheral or vascular venous tissue encountered with other routes of administration such as oral, intravenous, transdermal, and subcutaneous administration. The method can comprise the step of identifying and selecting an active agent which activity is metabolized or degraded by oral, subcutaneous or intravenous administration. This contrasts with peptides such as insulin which can be delivered effectively by such modes of administration. In these options, the method of administering a drug is advantageous for, for example, rapid onset of treatment since the drug can reach the target organ more rapidly through the arterial circulation without invasive therapy such as injections.

In certain options, the method of treatment of a disease or disorder comprises the step of selecting a suitable carrier for inhalation and delivering an active substance to pulmonary alveoli. The carrier can be associated with one or more active agents to form a drug/carrier complex which can be administered as a composition that avoids rapid degradation of the active agent in the peripheral and vascular venous tissue of the lung. In one option, the carrier is a diketopiperazine.

The method described herein can be utilized to deliver many types of active agents, including small molecules and biologicals. In particular options, the method utilizes a drug delivery system that effectively delivers a therapeutic amount of an active agent, including, small molecules or peptide hormones, rapidly into the arterial circulation. The one or more active agents may include, but are not limited to peptides, proteins, lipokines, small molecule pharmaceuticals, nucleic acids and the like, which is/are sensitive to degradation or deactivation; formulating the active agent into a dry powder composition comprising a diketopiperazine and delivering the active agent(s) into the systemic circulation by pulmonary inhalation using a cartridge and a dry powder inhaler. In one option, the method comprises selecting a peptide that is sensitive to enzymes in the local vascular or peripheral tissue of, for example, the dermis, or lungs. The disclosed method allows the active agent to avoid or reduce contact with peripheral tissue, venous or liver metabolism/degradation. In another option, for systemic delivery the active agent should not have specific receptors in the lungs.

The drug delivery system can also be used to deliver therapeutic peptides or proteins of naturally occurring, recombinant, or synthetic origin for treating disorders or diseases, including, but not limited to adiponectin, cholecystokinin (CCK), secretin, gastrin, glucagon, motilin, somatostatin, brain natriuretic peptide (BNP), atrial natriuretic peptide (ANP), parathyroid hormone, parathyroid hormone related peptide (PTHrP), IGF-1, growth hormone releasing factor (GHRF), granulocyte-macrophage colony stimulating factor (GM-CSF), anti-IL-8 antibodies, IL-8 antagonists including ABX-IL-8; integrin beta-
4 precursor (ITB4) receptor antagonist, enkephalins, nociceptin, nocistatin, orphanin FQ2, calcitonin, CGRP, angiotensin, substance P, neurokinin A, pancreatic polypeptide, neuropeptide Y, delta-sleep-inducing peptide, prostaglandins including PG-12, LTB receptor blockers including, LY29311, BIIL 284, CP105696; vasoactive intestinal peptide; triptans such as sumatriptan and lipokines such as C16:1n7 or palmitoleate or analogs thereof. In yet another option, the active agent is a small molecule drug,

In some options, the dry powder formulation is a stable composition and can comprise microparticles which are suitable for inhalation and which dissolve rapidly in the lung and rapidly deliver a drug, such as a serotonin receptor agonist, to the pulmonary circulation. Suitable particle sizes for pulmonary administration can be less than 10 µm in diameter, and preferably less than 5 µm. Exemplary particle sizes that can reach the pulmonary alveoli range from about 0.5 µm to about 5.8 µm in diameter. Such sizes refer particularly to aerodynamic diameter, but often also correspond to actual physical diameter as well. Such particles can reach the pulmonary capillaries and can avoid extensive contact with the peripheral tissue in the lung. In this option, the drug can be delivered to the arterial circulation in a rapid manner and avoid degradation of the active ingredient by enzymes or other mechanisms prior to reaching its target or site of action in the body. Dry powder compositions for pulmonary inhalation comprising a serotonin receptor modulator such as a serotonin receptor agonist and FDKP can comprise microparticles wherein from about 35% to about 75% of the microparticles have an aerodynamic diameter of less than 5.8 µm.

The methods of delivery presented in various options can provide a more direct path to an active agent's site of action. Thus, in addition to the avoidance of degradation, though in some instances still in part due to it, the biodistribution of the active agent can differ from that achieved with modes of delivery that entail absorption into and travel through the venous circulation prior to reaching sites of action in the body. Thus, sampling of venous blood to determine active agent concentration may underestimate the concentration of active agent at a site of action when using options of the present disclosure while, in comparison, overestimating it when other modes of administration are used. The more labile an active agent is the greater this effect can be. For active agents with multiple effects and sites of action, a different constellation of effects may be observed as the relative concentrations at different sites of action will differ from that achieved using other modes of administration. This can further contribute to greater effective bioavailability, avoidance of unwanted effects and the like.

In one option, the inhalable formulation comprises a dry powder formulation comprising the a serotonin receptor agonist with a diketopiperazine, including 2,5-diketo-3,6-di(4-X-aminobutyl)piperazine; wherein X is selected from the group consisting of succinyl, glutaryl, maleyl, and fumaryl, or a salt of the diketopiperazine. The inhalable formulation can comprise microparticles for inhalation comprising the active ingredient with the aerodynamic characteristics as described above. The amount of active ingredient can be determined by one of ordinary skill in the art, however, the present microparticles can be loaded with various amounts of active ingredient as needed by the patient. For example, for a serotonin receptor agonist, the microparticles can comprise from about 1% (w/w) to about 75% (w/w) of the active ingredient in the formulation. The inhalable formulations can comprise from about 10% (w/w) to about 30% (w/w) of the pharmaceutical composition and can also comprise a pharmaceutically acceptable carrier, or excipient, such as a surfactant, such as polysorbate 80. In this option, a serotonin receptor agonist can be administered to the patient from once to about four times a day or as needed by the patient with doses ranging from about 0.05 mg up to about 5 mg in the formulation.

The formulation comprising the active ingredient can be administered to the patient in a dry powder formulation by inhalation using a dry powder inhaler such as the inhaler disclosed, for example, in U.S. Patent No. 7,305,986 and U.S. Patent Application Serial No. 10/655,153 (US 2004/0182387). Repeat inhalation of dry powder formulations comprising the active ingredient can also be administered as needed. The formulation can be administered once, twice, three or four times a day.

In still yet a further option, the method comprises the administration of an inhalable dry powder composition comprising a diketopiperazine having the formula 2,5-diketo-3,6-di(4-X-aminobutyl)piperazine, wherein X is selected from the group consisting of succinyl, glutaryl, maleyl, and fumaryl. The dry powder composition can comprise a diketopiperazine salt. In still yet another option, there is provided a dry powder composition, wherein the diketopiperazine is 2,5-diketo-3,6-di-(4-fumaryl-aminobutyl)piperazine, with or without a pharmaceutically acceptable carrier, or excipient.

There is also disclosed, a method for treating migraines using a therapeutically effective pharmaceutical composition comprising a powder for pulmonary delivery is disclosed, wherein the powder comprises microparticles of a diketopiperazine and an active agent such as a serotonin receptor agonist for treating migraines. In this option, the pharmaceutical composition comprises, for example, a diketopiperazine, including, FDKP or an FDKP salt, for example, a divalent salt of FDKP, including disodium FDKP, and a small molecule, including a vasoconstrictor as the active agent Examples of vasoconstrictors are serotonin receptor agonists including, tripans such as sumatriptan, almotriptan, eletriptan, frovatriptan, naratriptan, rizatriptan, zolmitriptan and pharmaceutically acceptable salts thereof, including sumatriptan succinate, rizatriptan benzoate, almotriptan malate. In one option, the vasoconstrictor, for example, a triptan can be provided to a patient in need of treatment in amounts ranging from at least about 0.1 mg, at least about 1 mg, at least about 5 mg, about 50 mg or less, about 40 mg or less, about 1 mg to about 50 mg, about 5 mg to about 30 mg, about 10 mg. to about 20 mg, about 1 mg, about 10 mg, about 20 mg, or any amount in a range bounded by, or between, any of these values. A pharmaceutical composition comprising a triptan may be given regularly, including daily, twice daily, thrice daily, etc., and/or may be given as need at the onset of migraine symptoms. The triptan can be administered to the patient by inhalation. In a particular option, the triptan is provided to a patient by oral inhalation for delivery to the arterial circulation in the lungs.

The drug delivery formulation can comprise an aliphatic amino acid, for example, alanine, glycine, leucine, isoleucine, norleucine, and serine. In certain options, the aliphatic amino acid is from about 0.5% to about 30% by weight of the composition. In a particular option, the pharmaceutical composition comprises L-leucine. In one option, the pharmaceutical composition comprises a dry powder for oral inhalation comprising FDKP disodium salt, sumatriptan and L-leucine.

### EXAMPLES

The following examples are included to demonstrate certain embodiments. It should be appreciated by those of skill in the art that the techniques disclosed in the examples elucidate representative techniques that function well in the practice of the present invention.

### EXAMPLE 1

### Preparation and characterization of Sumatriptan-Disodium Fumaryl Diketopiperazine (Sumatriptan-Na₂FDKP) Dry Powder

Sumatriptan-Na₂FDKP powder was prepared from commercially available tablets of sumatriptan succinate. Sumatriptan succinate (Imitrex^{®}, GlaxoSmithKline) was extracted from crushed tablets suspended in HPLC grade water to form a solution. The solution was then filtered through 0.45 µm nylon syringe filters to remove undissolved excipients and the filtrate was processed through a quaternary amine ion exchange extraction column to eliminate the succinate group. The sumatriptan in solution at a concentration greater than 10 mg/mL was then combined with a solution of Na₂FDKP at a concentration greater than about 10 mg/mL in solution. The Na₂FDKP was either prepared earlier or prepared from the FDKP free acid by dissolution with two equivalents of sodium hydroxide. In some experiments, the ratio of the concentration of sumatriptan starting solution to FDKP was greater than 1. The solution was spray dried (Büchi Mini Spray Dryer Model B-290) at an inlet temperature of from about 145 °C to about 200 °C and an outlet temperature of from about 75 °C to about 85 °C. The drying gas was nitrogen set at a flow rate of about 670 L/hr. A dried powder was obtained.

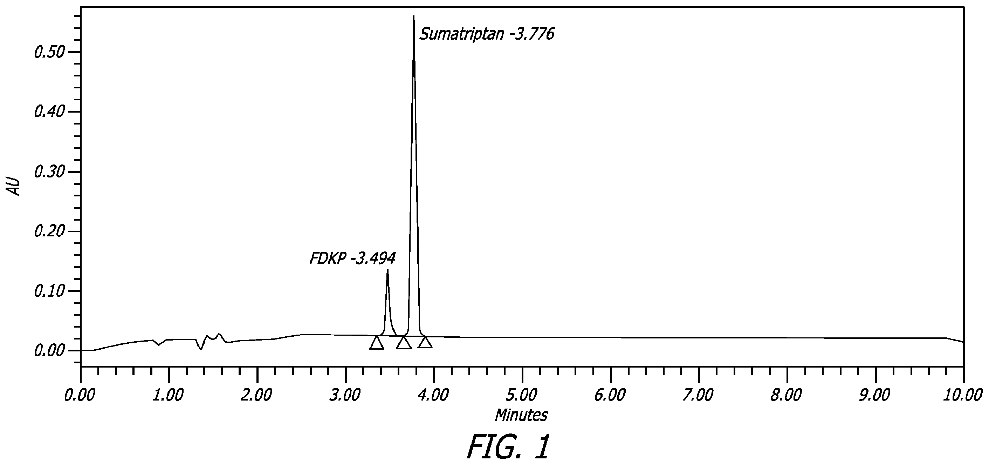

To ascertain the amount of sumatriptan in the powder, a sample of the powder was dissolved in an HPLC solution and assayed for content using a high pressure liquid chromatography (HPLC) system. The HPLC method quantifies sumatriptan in the presence of FDKP. FIG. 1 depicts an HPLC chromatogram of a solution of the dissolved powder, which shows that the two compounds can be easily separated and identified. Powders were also tested by thermal analysis (TGA and DSC) and cascade impaction. Table 1 shows representative data of Sumatriptan- Na₂FDKP powder characteristics made by the instant method, wherein the ratio of sumatriptan succinate concentration to FDKP in solution was about 1.5.

**Table 1**

| Assay (wt%) | | | | Cascade impaction | |
|---|---|---|---|---|---|
| Sumatriptan (% by weight) | FDKP | % LOD | Total | % RF/fill | % CE |
| 38.8* | 37.0 | 3.4 | 79.2 | 17.9 | 68.0 |

| | | | | | |
|---|---|---|---|---|---|
| *Target Sumatriptan level = 40% | | | | | |

Sumatriptan-Na₂FDKP powders made by the present method contained up to about 40% by weight of sumatriptan. The water content determined by loss on drying (LOO) was about 3.4%. The percent respirable fraction per fill was about 18%, and the amount of powder delivered by or emitted from the inhaler (cartridge emptying, CE) was about 68% using a using a breath powered inhaler as described in U.S. Patent Application Serial No. 12/473,125 (US 2009/0308390).

Table 2 illustrates the characterization of the bulk dry powder.

**Table 2**

| Assay (wt%) | | | | Cascade impaction | |
|---|---|---|---|---|---|
| Sumatriptan (% by weight) | FDKP | % LOD | Total | % RF/fill | % CE |
| 38.8* | 37.0 | 3.4 | 79.2 | 17.9 | 68.0 |

| | | | | | |
|---|---|---|---|---|---|
| *Target Sumatriptan level = 40% | | | | | |

### EXAMPLE 2

### Sumatriptan-Na₂FDKP Dry Powder - Pharmacokinetic (PK) and Pharmacodynamic (PD) Studies in Rats

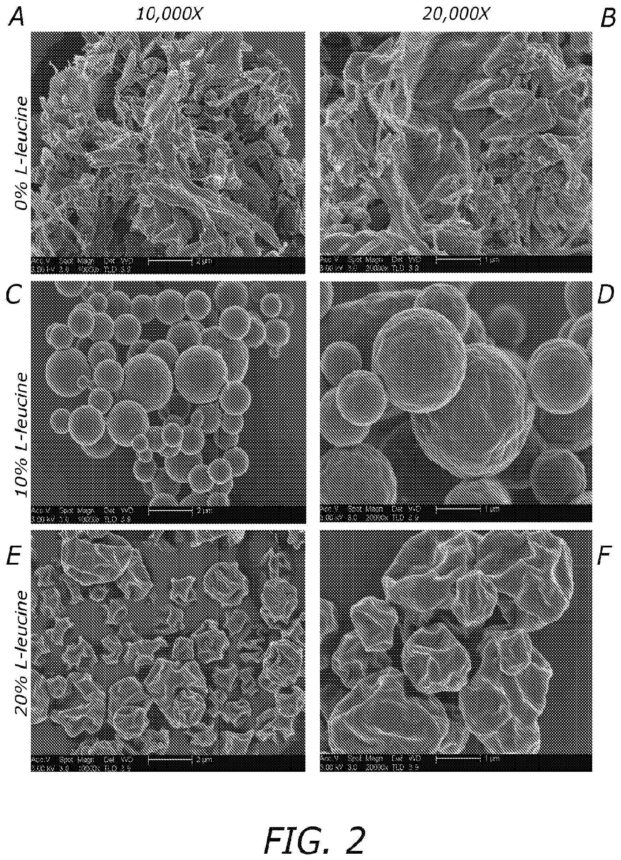

*Powder preparation and characterization:* Sumatriptan-Na₂FDKP powder was prepared as described in Example 1 above, except that the sumatriptan succinate was purchased from LGM Pharma (Boca Raton, FL) and L-leucine was added to study whether the aerodynamic performance of the resulting spray-dried powder formed would be improved. Three feed solutions were prepared at 4.5% total solids concentration for a 5 g scale. The feed solutions were prepared by adding FDKP disodium salt, sumatriptan succinate, and L-leucine (0 - 20 wt%) to de-ionized water with mixing. The solutions were titrated with dilute aqueous ammonia to pH 6.00. The resulting clear feed solutions were vacuum filtered through a 0.2 µm PES filter membrane and spray dried as described in Example 9, however, the drying gas flow was set at 25 kg/hr, the atomization flow was about 4 kg/hr and the atomization pressure was set at 4 bar. The sumatriptan succinate concentration (dry basis) in each solution was 56% to obtain a 40% sumatriptan target load. The powders were analyzed by HPLC, cascade impaction, Karl Fischer titration, scanning electron microscopy (SEM) and tap and bulk density. The results of these studies are shown in Table 2 and FIG. 2.

**Table 2**

| % L-leucine | Sumatriptan Assay (wt%) | %RF/fill | %CE | %water | Bulk density g/mL | Tapped density g/mL |
|---|---|---|---|---|---|---|
| 0 | 39.8 | 9.8 | 58.8 | 1.9 | 0.25 | 0.38 |
| 10 | 37.6 | 61.3 | 93.3 | 5.2 | 0.18 | 0.38 |
| 20 | 38.8 | 63.3 | 88.2 | 4.4 | 0.18 | 0.31 |

The data in Table 2 illustrate that the target and measured sumatriptan content for the bulk sumatriptan-Na₂FDKP powders are comparable. Aerodynamic performance improved with the addition of leucine. The powder without leucine had a %RF/fill of 9.8% with 58.8% CE, the addition of 10% leucine increased %RF/fill to 61.3% with 93.3% CE, and the addition of 20% leucine increased RF/fill to 63.3.% with 88.2% CE. The leucine-containing sumatriptan-Na₂FDKP powders had higher residual water content than the leucine-free powder. The addition of leucine also reduced the bulk powder density by approximately 30%.

FIG. 2 is a scanning electron micrograph of the three powders characterized in Table 2. As shown in panels A - F, each powder had distinct morphology. The particles without leucine were fused fragments with no uniform shape (Panel A and B). The particles with 10% leucine (Panel C and D) were substantially spherical with smooth surfaces and the particles made with 20% leucine (Panel E and F) have a raisin-like or shriveled morphology, typical of insulin- FDKP salt powders.

*Stability of Sumatriptan- Na*₂*FDKP powders:* Powders were also tested to determine their degree of stability. Samples of powder were incubated for a period of three months in an open dish exposed at 25 °C/60% relative humidity (RH) and at 40 °C/70% RH. Samples of the powders were assay by the HPLC method at 1, 2 and 3 months after the start of the experiments. The results are presented in Tables 3 and 4 below.

**Table 3**

| | **Storage Condition: 25°C/60%RH** | | | |
|---|---|---|---|---|
| | **Sumatriptan Assay** | | | |
| | *Initial* | *1 month* | *2 months* | *3 months* |
| 0% L-leucine powder | 39.8 (100%) | 38.9% (98%) | 39.9% (100%) | 39.6% (98%) |
| 10% L-leucine powder | 37.6 (100%) | 39.1% (104%) | 40.0% (106%) | 39.0% (104%) |
| 20% L-leucine powder | 38.8 (100%) | 38.9% (100%) | 40.2% (104%) | 39.4% (102%) |

**Table 4**

| | **Storage Condition: 40°C/75%RH** | | |
|---|---|---|---|
| | **Sumatriptan Assay** | | |
| | *Initial* | *7 days* | *14 days* |
| 0% L-leucine powder | 39.8 (100%) | 38.0 (95%) | 37.8 (95%) |
| 10% L-leucine powder | 37.6 (100%) | 38.8 (103%) | 38.6 (103%) |
| 20% L-leucine powder | 38.8 (100%) | 39.3% (101%) | 39.3 (101%) |

The data show that there was no degradation of the sumatriptan in the composition even after three months of exposure to 25 °C/60% RH with or without L-leucine. At higher temperature, 40 °C/70% RH, however, an insignificant, but slight decrease in sumatriptan content is observed after 1 and 2 weeks of incubation when compared to the samples containing L-leucine.

*Inhalation studies in rats using* Sumatriptan-Na₂FDKP *powders:* Powders prepared as described above were used in these experiments. The PK profile of sumatriptan administered as sumatriptan-Na₂FDKP powder (37.4% sumatriptan by weight) by pulmonary insufflation was evaluated and compared to sumatriptan nasal spray administered by pulmonary instillation or sumatriptan administered by intravenous injection or subcutaneous injection in female Sprague Dawley rats (n=6/group) (Table 5).

**Table 5**

| Group | Test Article | Achieved Sumatriptan Dose (mg) |
|---|---|---|
| 1 | Air Control | 0 |
| 2 | Sumatriptan b intravenous injection | 0.358 |
| 3 | Sumatriptan b subcutaneous injection | 0.358 |
| 4 | Sumatriptan nasal spray by pulmonary instillation | 0.483 |
| 5 | Sumatriptan-Na₂FDKP powder by pulmonary insufflation | 0.169* |

| | | |
|---|---|---|
| * mean achieved dose | | |

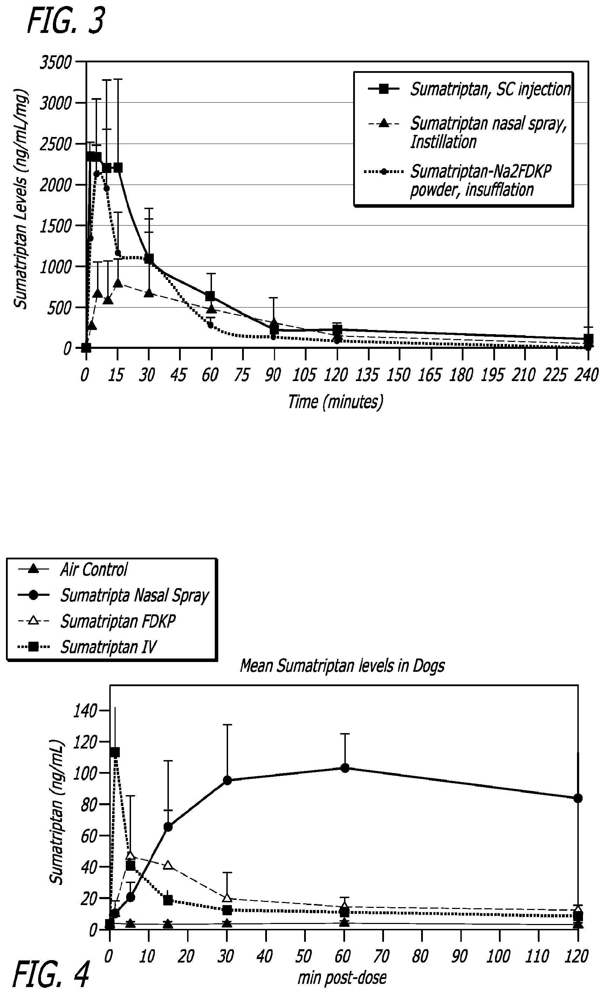

Blood samples for sumatriptan analysis were collected before dosing and at 2, 5, 10, 15, 30, 60, 90, 120 and 240 minutes after dosing. Animals were divided into two subsets (n = 3/timepoint) for blood collection. Sumatriptan in serum was analyzed using an established LCMS assay. Maximum concentration and bioavailability of sumatriptan insufflated as sumatriptan-Na₂FDKP powder was higher than the sumatriptan administered by liquid instillation (nasal spray formulation) and comparable to sumatriptan administered by subcutaneous injection (Figure 3). FIG. 3 shows that the time to maximum concentration was 5 minutes in the insufflation group versus 15 minutes in the pulmonary liquid instillation group. Overall dose-normalized exposure was similar for sumatriptan pulmonary insufflation and pulmonary liquid instillation, but the PK profiles are quite different. Sumatriptan was well tolerated across all treatment groups. Pharmacokinetic parameters were calculated using noncompartmental methods and the nonlinear regression program WinNonlin v5.2 (Table 5) based on the mean concentration curve (n = 3/time point/formulation) after correction for the actual administered dose. Table 6 summarizes representative pharmacokinetic data in female Sprague Dawley rats.

**Table 6**

| Group | Route | AUC | Cₘₐₓ | tₘₐₓ | t_{1/2} | Bioavailability |
|---|---|---|---|---|---|---|
| | | (min,ng/mL)mg | ng/mL/mg | min | min | % |
| 2 | IV | 273,729 | 26,719 | 2 | 304 | 100 |
| 3 | SC | 99,583 | 2309 | 5 | 36.6 | 36.4 |
| 4 | LIS | 70,063 | 776 | 15 | 45.8 | 25.6 |
| 5 | INS | 69,411 | 2145 | 5 | 24 | 25.4 |

| | | | | | | |
|---|---|---|---|---|---|---|
| *IV=Intravenous injection; SC=subcutaneous injection; LIS=Pulmonary liquid instillation; INS=pulmary insufflation | | | | | | |

It is apparent that the bioavailability of sumatriptan administered as Na₂FDKP sumatriptan powder by pulmonary insufflation was comparable to sumatriptan nasal spray administered by liquid instillation, but its PK profile (tₘₐₓ, Cₘₐₓ) resembled SC injection.

### EXAMPLE 3

### Sumatriptan-Na₂FDKP Dry Powder - PK and PD Studies in Beagle Dogs

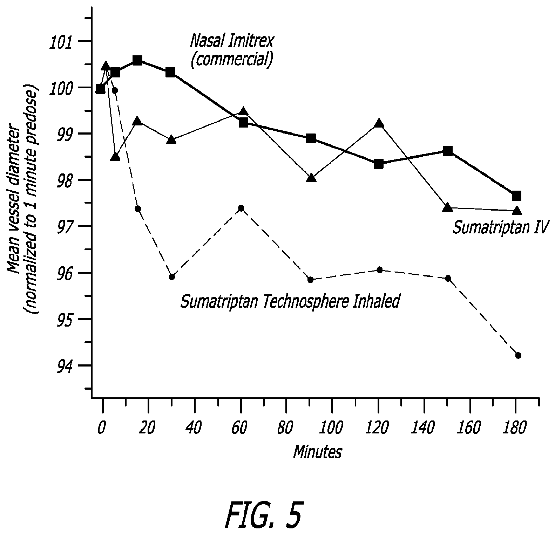

Pharmacodynamic Study: The pharmacodynamic and pharmacokinetic profiles of sumatriptan were evaluated in an accepted migraine model in anesthetized dogs. The pathogenesis of migraine is primarily due to a marked and prolonged period of vasodilation of cranial vessels. A model of migraine was induced by a single intra-arterial injection of capsaicin which produces carotid vasodilation. Animals received either air control (n = 2), sumatriptan by intranasal instillation using a microsprayer (0.28 mg/kg; n = 3), sumatriptan-Na₂FDKP dry powder by pulmonary insufflation (0.28 mg/kg sumatriptan; n = 3) or sumatriptan by intravenous bolus injection into a peripheral vessel (0.03 mg/kg; n = 2). Heart rate, systolic, diastolic, mean arterial blood pressures, and carotid blood flow and diameter (mean, maximum, minimum flow) were monitored and recorded continuously. Data were collected continuously and reported as 1-minute averages at specific time points after sumatriptan administration. The study summary is presented in Tables 7 and the results are shown in FIG. 5.

**Table 7. Experimental design**

| Group No. | No. of Animals | Model Induction | Test Material | Dose Levels (mg/kg, | Dose Regimen^{a.b} | Monitoring Period |
|---|---|---|---|---|---|---|
| 1 | 2 | Capsaicin administered intra-arterially via a 1 minute infusion | Control | 0 | Intra-tracheal (insufflator) | 30 minutes of stable baseline followed by 5 minutes of monitoring after administration of capsaicin and prior to sumatriptan administration. |
| 2 | 3 | | Sumatriptan^{C} | 0.28 | Nasal spray (microsprayer) | |
| 3 | 3 | | Sumatriptan Na₂FDKP powder | 0.29d | Intra-tracheal (insufflator) | |
| | | (56 µg/min; 1 mL/min) | | | | Monitoring continued through at least 3 hours after dosing with test article |
| 4 | 2 | | Sumatriptan^{c} | 0.030 | Intravenous | |

| | | | | | | |
|---|---|---|---|---|---|---|
| ^{a} Animals were anesthetized during all dosing procedures. ^{b} Dosing with the appropriate test article commenced 5 minutes after model induction. ^{c} Commercially available product (Imitrex^{®}). ^{d} Equivalent to 0.75 mg/kg powder dose based on 38% content of active ingredient Sumatriptan-Na₂FDKP powder. | | | | | | |

Blood samples from the dogs for sumatriptan analysis were collected before dosing and at 2, 5, 10, 15, 30, 60, 90, 120 and 240 minutes after dosing. Sumatriptan in serum was analyzed using an established LCMS assay.

Based on the PK data, one animal in the sumatriptan-Na₂FDKP powder group-treated appeared not to have not received test article, presumably due to technical difficulties. This animal showed unusually marked vasoconstriction which was suspect. Another animal in this group showed poor vasoconstriction and high levels of sumatriptan exposure. It was assumed that the tubes for blood samples from these two animals were inadvertently switched during collection. Therefore, data presented herewith were evaluated with (n = 3) and without (n = 2) the mis-dosed animal. Both sets of data suggest similar results.

Blood pressure and heart rate were unaltered by the administration of sumatriptan or control article, regardless of the route of administration. Systemic exposure of sumatriptan was associated with reductions in vasodilation. All groups, including the control, had reduction in carotid artery diameters from the end of capsaicin administration through 3 hours after dosing. Insufflation of sumatriptan-Na₂FDKP powder resulted in a more pronounced constriction of the carotid artery than the intra-nasal and intravenous routes of administration. The magnitude of vasoconstriction varied significantly between dose groups, so the data were analyzed in terms of vessel diameter relative to baseline diameter, or as a change in vessel diameter from the end of capsaicin dosing, or from baseline.

The pharmacokinetic profiles of sumatriptan administered as sumatriptan-Na₂FDKP dry powder, nasal spray, or intravenous injection ( FIG. 4 ) were consistent with the previous PK study in rats. FIG. 4 depicts the pharmacokinetic profile of sumatriptan FDKP salt powder (38% sumatriptan) administered by pulmonary insufflation, sumatriptan administered by nasal instillation, and intravenous injection in female dogs wherein the data are plotted as ± SD. The data show that the time to maximum mean peak circulating sumatriptan concentrations (Tₘₐₓ) was 5 minutes for the sumatriptan-FDKP salt powder and 60 minutes for nasal instillation. Even though Cₘₐₓ and bioavailability were much lower for the sumatriptan-Na₂FDKP dry powder, animals insufflated with sumatriptan-Na₂FDKP exhibited a similar but faster pharmacodynamic response than those receiving the nasal spray.

FIG. 5 shows results from these experiments. The data indicate that reduction in vessel diameter from the end of capsaicin to the end of experiment was largest in the group treated with the sumatriptan-Na₂FDKP powder. The variability in initial vasodilation between groups complicates the analysis, but Group 2 (nasal spray) and group 3 (sumatriptan-Na₂FDKP dry powder) responded comparably to capsaicin. The group treated with sumatriptan-Na₂FDKP dry powder experienced a larger net constriction in blood vessels and, moreover, the effect had a faster onset of action.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or options disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

## Claims

1. A dry powder inhaler comprising an inhalable pharmaceutical composition comprising a dry powder comprising microparticles of 3,6-bis[(N-fumaryl-4-aminobutyl)]-2,5-diketopiperazine or a salt thereof, and a triptan selected from sumatriptan, almotriptan, eletriptan, frovatriptan, naratriptan, rizatriptan, zolmitriptan and pharmaceutically acceptable salts thereof, wherein the pharmaceutical composition further comprises an aliphatic amino acid selected from the group consisting of alanine, glycine, leucine, isoleucine, norleucine and serine.

2. The dry powder inhaler of claim 1, wherein the triptan is present in an amount of 1-50 mg.

3. The dry powder inhaler of claim 1 or claim 2, wherein the aliphatic amino acid comprises 0.5% to 30% by weight of the pharmaceutical composition.

4. The dry powder inhaler of any one of claims 1-3, wherein the dry powder comprises microparticles, and 35% to 75% of the microparticles have an aerodynamic diameter of less than 5.8 µm.

5. The dry powder inhaler of any one of claims 1-4, wherein the pharmaceutical composition further comprises L-leucine.

6. A dry powder inhaler comprising an inhalable pharmaceutical composition comprising a dry powder comprising microparticles of 3,6-bis[(N-fumaryl-4-aminobutyl)]-2,5-diketopiperazine or a salt thereof, sumatriptan or rizatriptan, and 0.5% to 30% leucine or isoleucine.

7. The dry powder inhaler of claim 6, wherein the sumatriptan or rizatriptan is present in an amount of 1-50 mg.

8. The dry powder inhaler of any one of claims 1-7, wherein the 3,6-bis[(N-fumaryl-4-aminobutyl)]-2,5-diketopiperazine salt is an amorphous powder.

9. The dry powder inhaler of any one of claims 1-8, wherein the 3,6-bis[(N-fumaryl-4-aminobutyl)]-2,5-diketopiperazine is a crystalline powder.

10. The dry powder inhaler of any one of claims 1-9, wherein the rizatriptan is rizatriptan benzoate.

11. The dry powder inhaler of any one of claims 1-10, wherein the pharmaceutical composition further comprises a pharmaceutically acceptable excipient.

12. The dry powder inhaler of any one of claims 1-11, wherein the pharmaceutical composition further comprises a second medication or drug.

13. The dry powder inhaler of claim 12, wherein the second medication or drug is fluoxetine or duloxetine.

## Patentansprüche

1. Trockenpulverinhalator, umfassend eine inhalierbare pharmazeutische Zusammensetzung, die ein trockenes Pulver umfasst, das Mikropartikel von 3,6-Bis-[(N-fumaryl-4-aminobutyl)]-2,5-diketopiperazin oder einem Salz davon und ein Triptan, ausgewählt aus Sumatriptan, Almotriptan, Eletriptan, Frovatriptan, Almotriptan, Eletriptan, Frovatriptan, Naratriptan, Rizatriptan, Zolmitriptan und pharmazeutisch unbedenklichen Salzen davon, wobei die pharmazeutische Zusammensetzung ferner eine aliphatische Aminosäure, ausgewählt aus der Gruppe, bestehend aus Alanin, Glycin, Leucin, Isoleucin, Norleucin und Serin, umfasst.

2. Trockenpulverinhalator nach Anspruch 1, wobei das Triptan in einer Menge von 1-50 mg vorhanden ist.

3. Trockenpulverinhalator nach Anspruch 1 oder 2, wobei die aliphatische Aminosäure 0,5 bis 30 Gew.-% der pharmazeutischen Zusammensetzung umfasst.

4. Trockenpulverinhalator nach einem der Ansprüche 1 bis 3, wobei das Trockenpulver Mikropartikel umfasst und 35 % bis 75 % der Mikropartikel einen aerodynamischen Durchmesser von weniger als 5,8 µm aufweisen.

5. Trockenpulverinhalator nach einem der Ansprüche 1 bis 4, wobei die pharmazeutische Zusammensetzung ferner L-Leucin umfasst.

6. Trockenpulverinhalator, umfassend eine inhalierbare pharmazeutische Zusammensetzung, die ein trockenes Pulver umfasst, das Mikropartikel von 3,6-Bis[ (N-fumaryl-4-aminobutyl)]-2,5-diketopiperazin oder einem Salz davon, Sumatriptan oder Rizatriptan und 0,5 % bis 30 % Leucin oder Isoleucin umfasst.

7. Trockenpulverinhalator nach Anspruch 6, wobei das Sumatriptan oder Rizatriptan in einer Menge von 1-50 mg vorhanden ist.

8. Trockenpulverinhalator nach einem der Ansprüche 1 bis 7, wobei das 3,6-Bis[(N-fumaryl-4-aminobutyl)]-2,5-diketopiperazin-Salz ein amorphes Pulver ist.

9. Trockenpulverinhalator nach einem der Ansprüche 1 bis 8, wobei das 3,6-Bis[(N-fumaryl-4-aminobutyl)]-2,5-diketopiperazin ein kristallines Pulver ist.

10. Trockenpulverinhalator nach einem der Ansprüche 1 bis 9, wobei das Rizatriptan Rizatriptanbenzoat ist.

11. Trockenpulverinhalator nach einem der Ansprüche 1 bis 10, wobei die pharmazeutische Zusammensetzung ferner einen pharmazeutisch unbedenklichen Hilfsstoff umfasst.

12. Trockenpulverinhalator nach einem der Ansprüche 1 bis 11, wobei die pharmazeutische Zusammensetzung ferner ein zweites Medikament oder einen zweiten Wirkstoff umfasst.

13. Trockenpulverinhalator nach Anspruch 12, wobei das zweite Medikament oder Arzneimittel Fluoxetin oder Duloxetin ist.

## Revendications

1. Inhalateur de poudre seche comprenant une composition pharmaceutique inhalable comprenant une poudre seche comprenant des microparticules de 3,6-bis[(N-fumaryl-4-aminobutyl)]-2,5-dicétopipérazine ou un sel de celle-ci, et un triptan choisi parmi le sumatriptan, l'almotriptan, l'élétriptan, le frovatriptan, le naratriptan, le rizatriptan, le zolmitriptan et leurs sels pharmaceutiquement acceptables, dans lequel la composition pharmaceutique comprend en outre un acide aminé aliphatique choisi dans le groupe constitué par l'alanine, la glycine, la leucine, l'isoleucine, la norleucine et la sérine.

2. Inhalateur de poudre seche selon la revendication 1, dans lequel le triptan est présent en une quantité de 1 à 50 mg.

3. Inhalateur de poudre seche selon la revendication 1 ou la revendication 2, dans lequel l'acide aminé aliphatique comprend 0,5 % à 30 % en poids de la composition pharmaceutique.

4. Inhalateur de poudre seche selon l'une quelconque des revendications 1 à 3, dans lequel la poudre seche comprend des microparticules, et 35 % à 75 % des microparticules ont un diamètre aérodynamique inférieur à 5,8 µm.

5. Inhalateur de poudre seche selon l'une quelconque des revendications 1 à 4, dans lequel la composition pharmaceutique comprend en outre de la L-leucine.

6. Inhalateur de poudre seche comprenant une composition pharmaceutique inhalable comprenant une poudre seche comprenant des microparticules de 3,6-bis[(N-fumaryl-4-aminobutyl)]-2,5-dicétopipérazine ou un sel de celle-ci, de sumatriptan ou de rizatriptan, et de 0,5 % à 30 % de leucine ou d'isoleucine.

7. Inhalateur de poudre seche selon la revendication 6, dans lequel le sumatriptan ou le rizatriptan est présent en une quantité de 1 à 50 mg.

8. Inhalateur de poudre seche selon l'une quelconque des revendications 1 à 7, dans lequel le sel de 3,6-bis[(N-fumaryl-4-aminobutyl)]-2,5-dicétopipérazine est une poudre amorphe.

9. Inhalateur de poudre seche selon l'une quelconque des revendications 1 à 8, dans lequel la 3,6-bis[(N-fumaryl-4-aminobutyl)]-2,5-dicétopipérazine est une poudre cristalline.

10. Inhalateur de poudre seche selon l'une quelconque des revendications 1 à 9, dans lequel le rizatriptan est le benzoate de rizatriptan.

11. Inhalateur de poudre seche selon l'une quelconque des revendications 1 à 10, dans lequel la composition pharmaceutique comprend en outre un excipient pharmaceutiquement acceptable.

12. Inhalateur de poudre seche selon l'une quelconque des revendications 1 à 11, dans lequel la composition pharmaceutique comprend en outre un second traitement ou médicament.

13. Inhalateur de poudre seche selon la revendication 12, dans lequel le second traitement ou médicament est la fluoxétine ou la duloxétine.
